**Europäisches Patentamt**

(19) **European Patent Office**

**Office européen des brevets**

(11) Veröffentlichungsnummer: **0 002 533**
**B1**

(12) **EUROPÄISCHE PATENTSCHRIFT**

(45) Veröffentlichungstag der Patentschrift:
25.06.86

(51) Int. Cl.⁴: **A 61 K 31/215**, A 61 K 31/07,
A 61 K 31/165

(21) Anmeldenummer: **78101701.7**

(22) Anmeldetag: **15.12.78**

(54) **Verwendung von Retinoiden als Arzneimittel.**

(30) Priorität: **21.12.77 CH 15795/77**

(43) Veröffentlichungstag der Anmeldung:
**27.06.79 Patentblatt 79/13**

(45) Bekanntmachung des Hinweises auf die Patenterteilung:
**25.06.86 Patentblatt 86/26**

(84) Benannte Vertragsstaaten:
**BE CH DE FR GB IT LU NL SE**

(56) Entgegenhaltungen:
**AT - B - 300 189**
**AT - B - 340 902**
**AT - B - 340 903**
**CH - A - 257 577**
**DE - A - 2 050 658**
**DE - A - 2 542 366**
**FR - A - 2 059 480**
**FR - A - 2 223 037**
**FR - A - 2 285 864**
**FR - A - 2 331 331**
**FR - A - 2 374 291**
**FR - M - 653**
**NL - A - 7 612 200**

**THE MERCK INDEX, 1976, E. Merck & Co., Rahway, N.J.,
USA**

(73) Patentinhaber: **F. HOFFMANN-LA ROCHE & CO.
Aktiengesellschaft, CH-4002 Basel (CH)**

(72) Erfinder: **Bollag, Werner, Dr., Mythenstrasse 10,
CH-4054 Basel (CH)**
Erfinder: **Reber, Kurt, Dr., Eigenweg 42,
CH-4107 Ettingen (CH)**

(74) Vertreter: **Lederer, Franz, Dr. et al, Patentanwälte
Lederer, Meyer Lucile-Grahn-Strasse 22,
D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es wurde gefunden, dass Retinoide, nämlich Vitamin A-Säure, 13-cis-Vitamin-A-Säure,

9-(4-Methoxy-2,3,6-trimethylphenyl)-3,7-dimethyl-nona-2,4, 6,8-tetraen-1-säureäthylamid,

9-(2,6-Dichlor-5-methoxy-5-methyl-phenyl)-3,7-dimethyl-nona-2,4,6,8-tetraen-1-säureäthylester,

4-O-α-D-Glucopyranosyl-D-glucopyranosyl-9-(4-methoxy-2,3,6-trimethyl-phenyl)-3,7-dimethyl-nona-2,4,6,8-tetraenat,

3,7-Dimethyl-9-(2,4,5-trimethyl-3-thienyl)-nona-2,4,6,8-tetraensäureäthylester,

9-(4-Methoxy-2,3,6-trimethylphenyl)-4-fluoro-3,7-dimethyl-nona-2,4,6,8-tetraen-säureäthylester

und insbesondere

9-(4-Methoxy-2,3,6-trimethylphenyl)-3,7-dimethyl-nona-2,4,6,8-tetraen-1-säureäthylester,

bei oraler Verabreichung rheumatische Erkrankungen günstig beeinflussen. Es ist bekannt, diese Verbindungen in der topischen und systemischen Therapie von Neoplasien, Akne, Psoriasis und anderen dermatologischen Affektionen zu verwenden (siehe z.B. FR-A-2 223 037, DE-A-2 542 366, FR-A-2 059 480, AT-B-300 189 und FR-A-2 331 331).

Überraschend wurde nun gefunden, dass durch orale Verabreichung der oben genannten Retinoide andere pathologische Zustände als die oben erwähnten, nämlich rheumatische Erkrankungen, erfolgreich behandelt werden können.

Die Erfindung betrifft die neue Verwendung der oben genannten Retinoide, insbesondere des 9-(4-Methoxy-2,3,6-trimethyl-phenyl)-3,7-dimethyl-nona-2,4,6,8-tetraen-1-säureäthyl-esters, zur Herstellung von Mitteln zur oralen Behandlung rheumatischer Erkrankungen, insbesondere solcher entzündlicher und degenerativer Art, die Gelenke, Muskeln, Sehnen und andere Teile des Bewegungsapparates befallen. Beispiele solcher Erkrankungen sind die primär chronische Polyarthritis, die Spondylarthritis ankylopoetica Bechterew und die Arthropathia psoriatica.

Zur Behandlung dieser Erkrankungen wird z.B. der 9-(4-Methoxy-2,3,6-trimethylphenyl)-3,7-dimethyl-nona-2,4, 6,8-tetraen-1-säureäthylester oral zweckmässig in einer Dosierung von etwa 0,25–2 mg/kg Körpergewicht pro Tag, vorzugsweise 0,5–1 mg/kg/Tag, verabreicht.

Die Dosis kann als Einzeldosis oder auf mehrere Teildosen verteilt verabreicht werden.

Als Verabreichungsform kommen die für orale Verabreichung üblichen Gebrauchsformen in Betracht, wie Kapseln, Dragées oder Tabletten. Die Herstellung solcher Gebrauchsformen kann in üblicher Weise, z.B. anhand der nachstehenden Beispiele erfolgen.

Beispiel 1
Kapsel zur oralen Verabreichung

| | | |
|---|---:|---|
| Wirkstoff | 25 | mg |
| Lactose | 52 | mg |
| Maisstärke | 20 | mg |
| mikrokristalline Cellulose | 40 | mg |
| Talk | 2,5 | mg |
| Magnesiumstearat | 0,5 | mg |

Der Wirkstoff wird auf übliche Weise mit den Hilfsstoffen gemischt und in Gelatinesteckkapseln der Grösse No. 4 von Hand oder maschinell abgefüllt.

Beispiel 2
Kapsel zur oralen Verabreichung

| | | |
|---|---:|---|
| Wirkstoff | 10 | mg |
| Lactose | 82 | mg |
| Maisstärke | 45 | mg |
| Talk | 2,5 | mg |
| Magnesiumstearat | 0,5 | mg |

Der Wirkstoff wird auf übliche Weise mit den Hilfsstoffen gemischt und in Gelatinesteckkapseln der Grösse No. 4 von Hand oder maschinell abgefüllt.

Beispiel 3
Kapsel zur oralen Verabreichung

| | | |
|---|---:|---|
| Wirkstoff | 5 | mg |
| Lactose | 102 | mg |
| mikrokristalline Cellulose | 30 | mg |
| Talk | 2,5 | mg |
| Magnesiumstearat | 0,5 | mg |

Der Wirkstoff wird auf übliche Weise mit den Hilfsstoffen gemischt und in Gelatinesteckkapseln der Grösse Nr. 4 von Hand oder maschinell abgefüllt.

Beispiel 4
Tabletten zur oralen Verabreichung

| | | |
|---|---:|---|
| Wirkstoff | 25 | mg |
| Lactose | 20 | mg |
| Maisstärke | 30 | mg |
| mikrokristalline Cellulose | 42 | mg |
| Talk | 2,5 | mg |
| Magnesiumstearat | 0,5 | mg |

Die Wirksubstanz wird mit Lactose gemischt und auf übliche Weise mit einem Maisstärkekleister granuliert. Anschliessend wird der Rest der Hilfsstoffe zugemischt und die Masse tablettiert. Danach werden die Tabletten auf übliche Weise mit einem wasserlöslichen wasserquellbaren Lack überzogen.

Beispiel 5
Tabletten zur oralen Verabreichung

| | | |
|---|---:|---|
| Wirkstoff | 10 | mg |
| Lactose | 30 | mg |
| Maisstärke | 30 | mg |
| mikrokristalline Cellulose | 47 | mg |
| Talk | 2,5 | mg |
| Magnesiumstearat | 0,5 | mg |

Die Wirksubstanz wird mit Lactose gemischt und auf übliche Weise mit einem Maisstärkekleister granuliert. Anschliessend wird der Rest der Hilfsstoffe zugemischt und die Masse tablettiert.

Danach werden die Tabletten auf übliche Weise mit einem wasserlöslichen oder wasserquellbaren Lack überzogen.

Beispiel 6

Tabletten zur oralen Verabreichung

| | |
|---|---|
| Wirkstoff | 5 mg |
| Lactose | 45 mg |
| Maisstärke | 30 mg |
| mikrokristalline Cellulose | 37 mg |
| Talk | 2,5 mg |
| Magnesiumstearat | 0,5 mg |

Die Wirksubstanz wird mit Lactose gemischt und auf übliche Weise mit einem Maisstärkekleister granuliert. Anschliessend wird der Rest der Hilfsstoffe zugemischt und die Masse tablettiert. Danach werden die Tabletten auf übliche Weise mit einem wasserlöslichen oder wasserquellbaren Lack überzogen.

Die Behandlungsdauer hängt von den individuellen Umständen ab, sie kann einige Wochen oder mehrere Monate betragen. Es kann erforderlich werden, die Dosierung während der Behandlung herabzusetzen, um unerwünschte Nebenwirkungen, wie Haarausfall oder Cheilitis, zu vermeiden. Die nachstehend aufgeführten Resultate der Behandlung der psoriatischen Arthropathie und der primär chronischen Polyarthritis zeigen die Wirksamkeit des 9-(4-Methoxy-2,3,6-trimethylphenyl)-3,7-dimethyl-nona-2,4, 6,8-tetraen-1-säureäthylesters

und können gleichzeitig als Richtlinie für die erfindungsgemässe Verwendung der Verbindung dienen.

In den Tabellen werden die Behandlungsresultate wie folgt bewertet:

| − | = | keine Besserung |
|---|---|---|
| + | = | mässige Besserung |
| + + | = | gute Besserung |

Der Articular-Index wurde nach Ritchie et al., Quart. J. Med. New Series XXXVII, No. 147 (1968), Seite 393 ff. bestimmt.

Tabelle I

Behandlung der psoriatischen Arthropathie
Dosierung: 1 mg/kg/Tag; Behandlungsdauer: 4 Wochen

| Patient Geschlecht/Alter | | Articular-Index | Morgensteifigkeit | funktioneller Status | Schmerz |
|---|---|---|---|---|---|
| ♂ | 35 | + | | + | + |
| ♂ | 28 | + + | | − | + + |
| ♂ | 20 | + + | | | |
| ♂ | 39 | + + | + + | − | + |
| ♀ | 43 | + + | + + | − | + |
| ♀ | 32 | + + | + + | − | + + |

Tabelle II

Behandlung der psoriatischen Arthropathie

| Patient Geschlecht/Alter | | Dosierung [mg/Tag] | Articular-Index | Morgensteifigkeit | funktioneller Status | Schmerz |
|---|---|---|---|---|---|---|
| ♂ | 20 | 35 (1 Monat) 25 (2 Monate) | + + | | | + + |
| ♀ | 51 | 60 (6 Monate) 35 (1 Woche) 25 (6 Wochen) | + + | + + | − | + |
| ♀ | 43 | 50 | + | | + | + |
| ♂ | 29 | 95 (4 Wochen) 100 (3 Wochen) 50 (7,5 Wochen) | + + | + + | − | + + |
| ♂ | 39 | 75 (8 Wochen) | + + | | − | + + |
| ♀ | 16 | 30 (9 Wochen) 20 (3 Wochen) | + + | | − | − |
| ♀ | 32 | 30 (6 Wochen) | + + | | + | + + |
| ♀ | 64 | 50 (13 Wochen) | + + | + + | + + | + + |
| ♀ | 56 | 70 (12 Wochen) 50 (6 Wochen) | + | + + | + | + |

Tabelle III

Behandlung der primär chronischen Polyarthritis
Dosierung: 1 mg/kg/Tag; Behandlungsdauer: 4 Wochen

| Patient Geschlecht/Alter | | Articular-Index | Morgensteifigkeit | funktioneller Status | Schmerz |
|---|---|---|---|---|---|
| ♀ | 64 | + | − | − | − |
| ♀ | 52 | + + | + + | − | + |
| ♀ | 40 | + | + + | − | + |
| ♀ | 75 | + + | + + | − | + |

Tabelle IV

Behandlung der primär chronischen Polyarthritis

| Patient Geschlecht/Alter | | Dosierung [mg/Tag] | Articular-Index | Griff-stärke | Morgen-steifigkeit | funktioneller Status | Schmerz |
|---|---|---|---|---|---|---|---|
| ♀ | 54 | 50 (14 Wochen) | + + | + | + + | + | + + |
| ♀ | 57 | 60 (4 Wochen) 50 (7 Wochen) 40 (3 Wochen) 50 (2 Wochen) | + | + | + | + | + |
| ♀ | | 50 (18 Wochen) | + | | | + | + |

Im Tierexperiment wurde die antiarthritische Wirkung der Substanz im Adjuvans-Arthritis-Modell wie folgt bestimmt:

Methode: Gruppen von 6 bis 8 Ratten erhalten eine intracutane Injektion von 0,1 ml komplettem Freund'schen Adjuvans. Unmittelbar vorher und 3 Wochen nachher wird der Durchmesser aller 4 Pfoten bestimmt. Als Messwert für das Einzeltier gilt die Summe der 4 Pfotendurchmesser. Die Versuchstiere werden, beginnend 3 Tage vor der Injektion des Adjuvans, während 2 Wochen mit den zu testenden Substanzen behandelt.

Auswertung: Es wird die prozentuale Änderung der Pfotendurchmesser der Testtiere ($\Delta T\%$) errechnet und in Prozent des entsprechenden Mittelwertes der Kontrolltiere ($\Delta K\%$) als relative Wirkung in % gemäss der Formel:

$$100 - \left(\frac{\Delta T\%}{\Delta K\%} \cdot 100\right) \%$$

ausgedrückt.

Die Resultate sind in der Tabelle V aufgeführt.

In einer weiteren Versuchsreihe wurde analog verfahren, die Behandlung jedoch erst 3 Wochen nach der Injektion des Adjuvans begonnen und dann 4 Wochen fortgesetzt. Die Resultate sind in Tabelle VI wiedergegeben.

Die Tabelle VII zeigt die Wirkung des 9-(4-Methoxy-2,3,6-trimethylphenyl)-3,7-dimethyl-nona,2,4, 6,8-tetraen-1-säureäthyl-esters (A), des entsprechenden Äthylamids (B) und der Vitamin A-Säure (C).

Tabelle V

| Dosis [mg/kg] | Anzahl Tiere | Pfotendurchmesser Versuchsbeginn | Versuchsende | Differenz | relative Wirkung % |
|---|---|---|---|---|---|
| − | 8 | 24,9 | 44,0 | 19,1 | − |
| 15 | 8 | 25,0 | 37,5 | 12,5 | 34,8 |
| 30 | 8 | 25,2 | 34,0 | 8,8 | 54,3 |
| 60 | 4 | 25,9 | 29,2 | 3,3 | 83,2 |

Tabelle VI

| Dosis [mg/kg] | Prozentualer Rückgang des Pfotenödems nach 1 Woche | 2 Wochen | 3 Wochen | 4 Wochen | Behandlungsdauer |
|---|---|---|---|---|---|
| − | −3* | 3 | 7,1 | 7,6 | |
| 60 | 12 | 21 | 25 | 28 | |

* d.h. Zunahme des Oedems

Tabelle VII

| Verbindung | Dosis [mg/kg] | Anzahl Tiere | Pfotendurchmesser Versuchsbeginn | Versuchsende | Differenz | relative Wirkung % |
|---|---|---|---|---|---|---|
| – | – | 8 | 24,8 | 44,1 | 19,3 | – |
| A | 30 | 8 | 24,8 | 33,0 | 8,2 | 57,4 |
| B | 30 | 8 | 24,5 | 34,0 | 9,5 | 50,0 |
| C | 30 | 8 | 24,6 | 34,2 | 9,6 | 49,9 |

**Patentanspruch**

Verwendung eines Retinoids einer der Formeln
9-(4-Methoxy-2,3,6-trimethylphenyl)-3,7-dimethyl-nona-2,4,6,8-tetraen-1-säure-äthylester,
Vitamin A-Säure, 13-cis-Vitamin A-Säure,
9-(4-Methoxy-2,3,6-trimethylphenyl)-3,7-
    dimethyl-nona-2,4,6,8-tetraen-1-säure-
    äthylamid,
9-(2,6-Dichlor-4-methoxy-5-methyl-phenyl)-
    3,7-dimethyl-nona-2,4,6,8-tetraen-1-säure-
    äthylester,
4-o-α-D-Glucopyranosyl-D-glucopyranosyl-
    9-(4-methoxy-2,3,6-trimethylphenyl)-3,7-
    dimethyl-nona-2,4,6,8,tetraenat,
3,7-Dimethyl-9-(2,4,5-trimethyl-3-thienyl)-
    nona-2,4,6,8-tetraensäure-äthylester und
9-(4-Methoxy-2,3,6-trimethyl-phenyl)-4-
    fluoro-3,7-dimethyl-nona-2,4,6,8-tetraen-
    säureäthylester,
zur Herstellung von Mitteln zur oralen Behandlung rheumatischer Erkrankungen.

**Claim**

The use of a retinoid of one of the formulae
9-(4-methoxy-2,3,6-trimethylphenyl)-3,7-
    dimethyl-nona-2,4,6,8-tetraen-1-oic acid
ethyl ester,
vitamin A acid, 13-cis-vitamin A acid,
9-(4-methoxy-2,3,6-trimethylphenyl)-3,7-
    dimethyl-nona-2,4, 6,8-tetraen-1-oic acid
    ethylamide,
9-(2,6-dichloro-4-methoxy-5-methyl-phenyl)-
    3,7-dimethyl-nona-2,4,6,8-tetraen-1-oic
    acid ethyl ester,
4-O-α-D-glucopyranosyl-D-glucopyranosyl-9-
    (4-methoxy-2,3,6-trimethyl-phenyl)-3,7-
    dimethyl-nona-2,4,6,8-tetraenate,
3,7-dimethyl-9-(2,4,5-trimethyl-3-thienyl)-
    nona-2,4,6,8-tetraenoic acid ethyl ester and
9-(4-methoxy-2,3,6-trimethyl-phenyl)-4-
    fluoro-3,7-dimethyl-nona-2,4,6,8-tetraenoic
    acid ethyl ester
for the manufacture of compositions for the oral
treatment of rheumatic diseases.

**Revendication**

Utilisation d'un rétinoïde répondant à l'une des
formules suivantes:
9-(4-méthoxy-2,3,6-triméthyl-phényl)-3,7-
    diméthyl-nona-2,4,6,8-tétraène-1-oate
d'éthyle,
acide de la vitamine A,
acide de la 13-cis-vitamine A,
éthylamide de l'acide 9-(4-méthoxy-2,3,6-tri-
    méthylphényl)-3,7-diméthyl-nona-2,4,6,8-
    tétraène-1-oïque,
9-(2,6-dichloro-4-méthoxy-5-méthylphényl)-
    3,7-diméthyl-nona-2,4,6,8-tétraène-1-oate
    d'éthyle,
4-o-alpha-D-glucopyrannosyl-D-glucopyran-
    nosyl-9-(4-méthoxy-2,3,6-triméthylphényl)-
    3,7-diméthyl-nona-2,4, 6,8-tétraénoate,
3,7-diméthyl-9-(2,4,5-triméthyl-3-thiényl)-
    nona-2,4,6,8-tétraénoate d'éthyle et
9-(4-méthoxy-2,3,6-triméthylphényl)-4-fluoro-
    3,7-diméthyl-nona-2,4,6,8-tétraénoate
d'éthyle,
pour la préparation d'agents pour le traitement
par voie orale de maladies rhumatismales.